# EUROPÄISCHE PATENTSCHRIFT

(11) **EP 4 142 820 B1**
(45) Veröffentlichungstag und Bekanntmachung des Hinweises auf die Patenterteilung: **18.02.2026**
(21) Anmeldenummer: 21717817.7
(22) Anmeldetag: 06.04.2021
(51) Int. Cl.: A61M 1/16, B01D 63/02, B29C 53/56

(54) **WICKELSYSTEM ZUR DURCHFÜHRUNG DES AUFWICKELNS VON VERKETTETEN HOHLFASERN AUF EINEN WICKELKERN**
WINDING SYSTEM FOR WINDING UP CONCATENATED HOLLOW FIBRES ONTO A WINDING CORE
SYSTÈME D'ENROULEMENT POUR EFFECTUER L'ENROULEMENT DE FIBRES CREUSES INTERCONNECTÉES SUR UN AXE D'ENROULEMENT

(30) Priorität: 30.04.2020 DE 102020111803
(43) Veröffentlichungstag der Anmeldung: 08.03.2023
(73) Patentinhaber: enmodes GmbH, 52068 Aachen (DE)
(72) Erfinder: RITTER, Ilse Philine, 52064 Aachen (DE); BORCHARDT, Ralf, 52066 Aachen (DE); KADEN, Jens, 52146 Würselen (DE)
(74) Vertreter: Cohausz Hannig Borkowski Wißgott
(86) Internationale Anmeldenummer: PCT/EP2021/058939
(87) Internationale Veröffentlichungsnummer: WO 2021/219330

(56) Entgegenhaltungen:
- EP-A1- 0 216 085
- EP-A1- 3 520 838
- WO-A1-97/33636
- FR-A5- 2 095 979
- US-A- 2 731 183
- US-A- 3 255 889
- US-A- 4 253 228

## Beschreibung

Die Erfindung betrifft ein Wickelsystem zur Durchführung des Aufwickelns von verketteten Hohlfasern, insbesondere von stoff- und/oder energiepermeablen Hohlfasern, auf einen Wickelkern, umfassend einen Wickelkern einer Austauschvorrichtung für den Stoff- und/oder Wärmeaustausch zwischen zwei Medien, insbesondere eines Oxygenators und/oder Wärmetauschers, auf den verkettete Hohlfasern aufwickelbar sind und der vorgesehen, insbesondere eingerichtet ist, zusammen mit den auf ihn aufgewickelten Hohlfasern in das Gehäuse der Vorrichtung eingesetzt zu werden und darin zu verbleiben.

Die Publikation EP 3 520 838 A1 zeigt ein Wickelsystem mit dem unverkettete Hohlfasern auf einen Wickelkern aufwickelbar sind. Es bleibt offen, ob mit der Vorrichtung auf den Wickelkern auch verkettete Hohlfasern aufwickelbar sind.

Im Stand der Technik ist es bekannt, stoff- oder energiepermeable Hohlfasern, insbesondere solche die zu Matten mit Kettfäden verbunden sind auf einen Kern zu wickeln, um das gewickelte Hohlfaserpaket in einer Austauschvorrichtung für den Stoff- und/oder Energieaustausch zwischen zwei Medien, insbesondere in einem Oxygenator und/oder einem Wärmetauscher, einzusetzen. Dies zeigen z.B. die Publikationen EP 0 089 122 A2 und EP 0 285 812 A1.

Hierfür wird das gewickelte Hohlfaserpaket in ein Gehäuse der Austauschvorrichtung eingesetzt und anschließend die Hohlfaserenden untereinander und mit dem Gehäuse verklebt, was auch als Verpottung bezeichnet wird. Innerhalb des Gehäuses können sodann nach Eröffnung der Hohlfaserenden, also nach der Entfernung der endseitigen Verklebung, zwei Medien durch die Hohlfaserwandungen separiert geführt werden, nämlich ein Medium, z.B. Blut außen an den Hohlfasern vorbei und ein anderes Medium, z.B. ein Gas oder Gasgemisch, oder ein temperiertes Fluid durch die Hohlfasern.

Über die Hohlfasern, insbesondere für einen Stoffaustausch über semipermeable Hohlfasern kann sodann ein Austausch stattfinden, z.B. in einem Oxygenator ein Austausch von Sauerstoff und Kohlendioxid. Solche gewickelten Hohlfaserpakete können auch in Stoffaustauschvorrichtungen für Dialysezwecke oder auch zur Kohlenmonoxid-Eliminierung eingesetzt werden.

Das Wickeln der Hohlfasern auf einen Kern und die Integration des erstellten Wickels in eine Austauschvorrichtung ist ein aufwändiger Prozeß und soll mittels der Erfindung vereinfacht werden. Insbesondere ist es eine weitere Aufgabe der Erfindung das Wickeln so vereinfacht zu gestalten, dass ein zum Aufwickeln genutzter Wickelkern einfach zum einen an einen Antrieb adaptiert werden kann, mit dem ein motorisches Aufwickeln erschlossen ist und andererseits auch in der Austauschvorrichtung direkt eingesetzt werden kann. Insbesondere ist es eine Aufgabe die Adaption des Wickelkerns an den Antrieb so zu gestalten, dass eine schnelle Adaption und rückstandslose Entfernung der Adaption möglich ist.

Gelöst wird diese Aufgabe durch ein Wickelsystem zur Durchführung des Aufwickelns von verketteten Hohlfasern, insbesondere von stoff- und/oder energiepermeablen Hohlfasern auf einen Wickelkern, umfassend einen Wickelkern einer Austauschvorrichtung für den Stoffaustausch und/oder Wärmeaustausch zwischen zwei Medien, insbesondere eines Oxygenators und/oder Wärmetauschers, auf den verkettete Hohlfasern aufwickelbar sind und der vorgesehen, insbesondere eingerichtet ist, zusammen mit den auf ihn aufgewickelten Hohlfasern in das Gehäuse der Vorrichtung eingesetzt zu werden und darin zu verbleiben und weiter erfindungsgemäß umfassend wenigstens ein Kupplungselement, vorzugsweise zwei Kupplungselemente, wobei das wenigstens eine Kupplungselement zwei sich axial gegenüberliegende Verbindungsbereiche aufweist und temporär mit dem ersten Verbindungsbereich mit dem Wickelkern drehfest verbindbar ist und mit dem zweiten Verbindungsbereich mit einem Antrieb drehfest verbindbar ist, mittels dem der Wickelkern um eine Wickelachse rotierbar ist, wobei der erste Verbindungsbereich des Kupplungselementes ein Spreizelement umfasst, insbesondere dessen Außenquerschnitt durch Aufspreizen vergrößerbar ist, und der Wickelkern in wenigstens einer axialen Stirnfläche, vorzugsweise in beiden axialen Stirnflächen eine um die Wickelachse angeordnete Ausnehmung aufweist, in welche der erste Verbindungsbereich eines Kupplungselementes hineinsteckbar und durch Aufspreizen des Spreizelementes der Wickelkern mit dem Kupplungselement drehfest verbindbar ist.

Der Kerngedanke des Wickelsystems beruht somit darauf, dass der mit dem Wickelsystem genutzte Wickelkern nach dem Aufwickeln der Hohlfasern bzw. der Hohlfasermatte(n) direkt in dem Gehäuse einer Austauschvorrichtung, z.B. einer Stoffaustauschvorrichtung als Träger der Hohlfasern verwendet werden kann und das zum Zweck des Aufwickelns, insbesondere eines motorischen Aufwickelns der Wickelkern auf einfache und schnelle Weise an einen Antrieb angekoppelt werden kann.

Hierfür nutzt das erfindungsgemäße Wickelsystem ein Kupplungselement oder vorzugsweise zwei um den Wickelkern herum auf einer gemeinsamen Drehachse gegenüberliegende Kupplungselemente, mit dem / denen die Adaption an einen Antrieb erfolgt. Dabei erfolgt die drehfeste Befestigung zwischen Wickelkern und Kupplungselement durch ein Aufspreizen des jeweiligen Kupplungselementes an seinem zum Wickelkern weisenden bzw. mit diesem zusammenwirkenden ersten Verbindungsbereich.

Ein solches Aufspreizen kann z.B. zwischen zwei Spreizstellungen erfolgen, wobei in der einen Spreizstellung die Verbindung zwischen Kupplungselement und Wickelkern gelöst ist und in der anderen Spreizstellung eine drehfeste Verbindung erzielt ist, so dass ein am zweiten Verbindungsbereich des Kupplungselementes angekoppelter Antrieb in der Lage ist, den Wickelkern zu rotieren und an den Wickelkern herangeführte Hohlfasermatten aufzuwickeln.

Soweit im Folgenden der Wickelkern und ein Kupplungselement weiter beschrieben wird, ist festzuhalten, dass die Beschreibung auch zutreffend ist, sofern am Wickelkern an beiden sich gegenüberliegenden axialen Stirnseiten / Stirnflächen jeweils ein Kupplungselement befestigt werden kann. Dies stellt ohnehin eine bevorzugte Ausführungsform dar. In diesem Fall gilt die Beschreibung für den Wickelkern hinsichtlich beider Stirnseiten / Stirnflächen und hinsichtlich jedes der beiden nutzbaren Kupplungselemente, insbesondere die identisch ausgeführt sein können.

Eine Spreizverbindung zwischen Wickelkern und Kupplungselement hat den Vorteil, dass die Verbindung erfolgt, ohne dass die beiden zu verbindenden Elemente sich axial, also translatorisch oder rotatorisch beim Herstellen der Verbindung zueinander bewegen.

Vielmehr wird bei der Spreizverbindung vorzugsweise erzielt, dass zusammenwirkende Kontaktflächen durch das Aufspreizen des Spreizelementes sich aufeinander zubewegen und in Kontakt zueinander gelangen, wodurch ein Reibschluß und/oder Kraftschluß erzielt wird.

Dies ist gegenüber anderen Verbindungsarten insoweit vorteilhaft, weil so ein Abrieb von Material zwischen den beiden Verbindungspartnern verhindert wird, wie er z.B. durch ein Einschrauben des Kupplungselementes in den Wickelkern zwischen diesen entstehen könnte. Ein solcher evtl. Abrieb kann negative Auswirkungen haben, wenn dieser durch Verwendung des Wickelkernes in einer Stoffaustauschvorrichtung in diese überführt wird und in den Blutkreislauf eines Patienten gelangen könnte. Darüber hinaus bietet die erfindungsgemäße Spreizverbindung den Vorteil durch Spritzgusstechnik hergestellt werden zu können.

Der Wickelkern, der im Wickelsystem zum Einsatz kommt und nach dem Aufwickeln in das Gehäuse einer Austauschvorrichtung eingesetzt wird und darin zu deren Betrieb verbleibt, kann z.B. dadurch für diesen Einsatz eingerichtet sein, dass der Wickelkern hinsichtlich seiner Geometrie und oder Abmessungen an das Gehäuse fertig angepasst ist, um ohne Änderung, außer dem Entfernen des Kupplungselementes oder der Kupplungselemente in das Gehäuse eingesetzt zu werden. Weiterhin kann vorzugsweise der Wickelkern für diesen Zweck auch wenigstens einen Kanal im Innerem des Wickelkerns aufweisen, durch den später im Betrieb ein Medium geführt werden kann.

Eine Einrichtung oder Eignung des Wickelkernes zu diesem Zweck kann auch in einer weiterhin als vorteilhaft angesehenen Ausführung darin liegen, dass eine Ausnehmung im Wickelkern (insbesondere beide Ausnehmungen) für die Aufnahme des Kupplungselementes gebildet ist durch einen den Wickelkern zumindest bereichsweise durchdringenden Kanal, der nach Einsetzen des Wickelkernes in das Gehäuse der Stoffaustauschvorrichtung von einem Medium durchströmbar ist, insbesondere von Blut. Ein solcher Kanal hat somit zwei Funktionen, nämlich sowohl in dem Wickelsystem zur Befestigung des Kupplungselementes als auch in der späteren Stoffaustauschvorrichtung für den Medientransport.

Der Kanal kann den Wickelkern beispielsweise axial vollständig durchdringen, insbesondere so dass zwei Kupplungselemente beidseitig am Wickelkern angesetzt werden können. Bei zwei Ausnehmungen zur Aufnahme von Kupplungselementen kann nur einer durch einen Kanal gebildet sein, der zum Medientransport dient, insbesondere der nicht vollständig axial durch den Wickelkern geführt ist.

Ein vorgenannter Kanal kann einseitig in die Ausnehmung zur Befestigung des Kupplungselementes münden und an seinem anderen Ende in einer oder mehreren Öffnungen auf der Mantelfläche des Wickelkerns münden, insbesondere wenn der Kanal nicht axial komplett durch den Wickelkern geht.

Ein durchgehender Kanal kann auch Abzweigungen aufweisen, die in die äußere Mantelfläche des Wickelkernes münden.

Durch Mündungsöffnungen in der Mantelfläche des Wickelkerns kann erzielt werden, dass im späteren Betrieb ein Medium, z.B. Blut durch diese Mündungsöffnungen in das gewickelte Hohlfaserpaket eindringen kann, vorzugsweise mit einer radialen Einströmkomponente.

Es kann weiterhin auch vorgesehen sein, dass eine (jeweilige) Ausnehmung im Wickelkern für die Aufnahme des Kupplungselementes umgeben ist von einem zur Ausnehmung zumindest bereichsweise koaxial verlaufenden Kanal, der nach Einsetzen des Wickelkernes in das Gehäuse der Stoffaustauschvorrichtung von einem Medium durchströmbar ist, insbesondere von Blut. Für einen solchen koaxialen Kanal können die vorgenannten Ausführungen in gleicher Weise zutreffen, insbesondere kann dieser also in die Mantelfläche des Wickelkerns münden oder Abzweigungen aufweisen, die darin münden.

Unabhängig von diesen vorgenannten Möglichkeiten eine Eignung des Wickelkerns für die Stoffaustauschvorrichtung zu erhalten kann es die Erfindung vorsehen, dass das wenigstens eine Kupplungselement an seinem ersten Verbindungsbereich einen Schaft aufweist der in axialer Richtung geteilt ist in wenigstens zwei axial parallel nebeneinanderliegende Schaftteile zwischen denen als Spreizelement ein axial zwischen die Schaftteile bewegliches Keilelement angeordnet ist. Bei einer bewirkten axialen Bewegung des Keilelementes zwischen die Schaftteile werden diese somit radial nach außen bewegt.

Vorzugsweise weist der Schaft wenigstens drei Schaftteile auf. Das Keilelement kann vorzugsweise konisch bzw. kegelabschnittförmig ausgebildet sein. Bei seiner axialen Bewegung kann zum Aufspreizen das Keilelement vorzugsweise vom ersten Verbindungsbereich in Richtung zum zweiten bewegbar sein. Um eine bewegende Kraft am Keilelement angreifen zu lassen kann das Keilelement durch einen hohlen Bereich des Schaftes geführt sein, z.B. in den zweiten Verbindungsbereich oder aus dem zweiten Verbindungsbereich heraus.

In einer anderen Ausführung kann es vorgesehen sein, dass das wenigstens eine Kupplungselement an seinem ersten Verbindungsbereich einen Schaft mit einem Schaftabschnitt aufweist, um den herum als Spreizelement eine Hülse aus einem elastischen Material, insbesondere aus einem Elastomer, vorzugsweise aus einem Vulkanisat von Silikonkautschuk oder Naturkautschuk, angeordnet ist, wobei die Hülse durch axiale Bewegung ihrer Stirnfläche zueinander, insbesondere durch Einwirkung einer Kraft in axialer Richtung auf die ringförmigen Stirnflächen der Hülse, in radialer Richtung in seiner Größe änderbar ist. Insbesondere kann es so vorgesehen sein, dass die Hülse im nicht verbundenen Zustand zwischen Kupplungselement und Wickelkern kraftfrei bzw. in relaxierten Zustand ist und zur Verbindung eine axiale Kraft ausgeübt wird, welche die axialen Stirnflächen der Hülse aufeinander zubewegt und sich hierdurch die Hülse radial verdickt.

Es kann in einer möglichen Ausführung vorgesehen sein, dass die Hülse mit einem ersten Ende ihrer beiden axialen Enden an einer zum Schaftbereich ortsfesten ersten Kraftausübungsfläche des Kupplungselementes anliegt und die Hülse mit dem zweiten axialen Ende an einer zweiten Kraftausübungsfläche des Kupplungselementes anliegt, wobei die zweite Kraftausübungsfläche relativ zur ersten Kraftausübungsfläche in axialer Richtung der Hülse, insbesondere auf dem Schaft beweglich ist, vorzugsweise mittels eines am Kupplungselement vorgesehenen Betätigungselementes. Die ortsfeste erste Kraftausübungsfläche kann an dem freien Ende des ersten Verbindungsbereiches angeordnet sein, der in die Ausnehmung des Wickelkern eingesteckt wird. Der Außenquerschnitt / Außendurchmesser der Kraftausübungsfläche ist vorzugsweise größer gleich dem Durchmesser der relaxierten Hülse an deren axialen Enden und kleiner gleich dem Innenquerschnitt / Innendurchmesser der Ausnehmung im Wickelkern.

Unabhängig von der Art der Aufspreizung kann es die Erfindung vorzugsweise vorsehen, dass in einem Zustand der Aufspreizung die Position des Kupplungselementes relativ zum Wickelkern axial fixierbar ist, vorzugsweise auch zu einem zum Kern separaten Hilfselement, das nach Einsetzen des ersten Verbindungsbereiches des Kupplungselementes in die Ausnehmung des Wickelkerns über das Kupplungselement herüber auf eine axiale Stirnfläche des Wickelkernes aufsetzbar ist, axial fixierbar ist.

Der Einsatz eines Hilfselementes zum Fixieren des Kupplungselementes in der Ausnehmung des Wickelkerns kann mehrere Vorteile haben.

Beispielsweise kann das Hilfselement, durch eine über den Wickelkern im Querschnitt/ Durchmesser in radialer Richtung überstehenden Scheibe ausgebildet sein. Eine solche Scheibe kann vorzugsweise eingesetzt sein, um beim Wickeln die Hohlfasermatte(n) relativ zum Wickelkern zu positionieren, insbesondere zu zentrieren, insbesondere wenn am Wickelkern beidseitig Kupplungselemente mit jeweiligen Scheiben befestigt sind, so dass eine aufgewickelte Hohlfasermatte sich beim Wickeln zwischen die Scheiben legt.

Weiterhin kann das Hilfselement für die Hülse eine zweite Kraftausübungsfläche bilden, die von der Hülse mit dem zweiten axialen Ende, insbesondere dem zum zweiten Verbindungsbereich des Kupplungselementes weisenden Ende der Hülse, kontaktierbar ist, wobei das zum zweiten Verbindungsbereich des Kupplungselementes weisende zweite Ende der Hülse im in den Wickelkern eingesetzten Zustand des Kupplungselementes über die axiale Stirnfläche des Wickelkernes hervorsteht und die Kraftausübung auf die Hülse durch das Aufsetzen des Hilfselementes bewirkt ist, insbesondere wodurch das zweite Ende der Hülse in Richtung zum ersten Ende der Hülse verlagerbar ist.

Ebenso kann es vorgesehen sein, dass das zum zweiten Verbindungsbereich des Kupplungselementes weisende zweite Ende der Hülse im in den Wickelkern eingesetzten Zustand des Kupplungselementes unter der axialen Stirnfläche oder in der Ebene der axialen Stirnfläche des Wickelkernes liegt und die Kraftausübung nach Aufsetzen des Hilfselementes durch eine axiale Bewegung das Kupplungselements, insbesondere eine aus dem Wickelkern heraus gerichtete Bewegung bewirkbar ist, insbesondere wodurch das erste Ende der Hülse in Richtung zum zweiten Ende der Hülse verlagerbar ist.

In beiden Fällen wird durch eine Zusammenwirkung des Hilfselementes bzw. der Scheibe mit einer der axialen Stirnflächen der Hülse eine Bewegung der beiden Stirnflächen der Hülse aufeinander zu erzeugt und damit die radiale Verdickung der Hülse.

Unabhängig von der Art der Ausführung des Spreizelementes kann es die Erfindung vorsehen, dass die Position des Kupplungselementes axial fixierbar ist, z.B. mittels einem in eine Ringnut am Kupplungselement einsetzbaren Sicherungsring, mit dem das Kupplungselement an der Oberfläche des Hilfselementes abstützbar ist.

Diese Ringnut kann vorzugsweise zwischen den beiden Verbindungsbereichen des Kupplungselementes liegen, insbesondere in einem Teilstück des Kupplungselementes, das in den Bereich übergeht, welcher das Spreizelement umfasst oder bildet, also vorzugsweise in den Schaftbereich übergeht, der die Hülse trägt oder der in das Keilelement übergeht.

Weiterhin kann in allen möglichen Ausführungen vorgesehen sein, dass am Kupplungselement ein Betätigungselement vorgesehen ist, mit dem die Aufspreizung des ersten Verbindungsbereiches und/oder die axiale Fixierung des Kupplungselementes bewirkbar ist, insbesondere das sich an dem Hilfselement oder einem die zweite Kraftausübungsfläche aufweisenden Element oder einem am Kupplungselement angeordneten Stützelement abstützt.

Ein Betätigungselement kann dabei z.B. ausgebildet sein als eine Mutter auf einem Gewindeabschnitt des Kupplungselementes, welcher mit dem Spreizelement verbunden ist. Der Gewindeabschnitt kann z.B. in den Schaftbereich übergehen, welcher die Hülse trägt oder in das Keilelement übergehen.

Ein Betätigungselement kann auch als ein mit einem Hebel bewegbarer Exzenter ausgebildet sein, der um eine Achse drehbar ist und sich am Kupplungselement oder dem Hilfselement abstützt, wobei der die Achse aufweisende Teil in den Schaftbereich übergeht, welcher die Hülse trägt oder in das Keilelement übergehen. Durch die exzentrische Bewegung kann so die Achse innerhalb des Kupplungselementes verlagert und das Spreizelement betätigt werden, insbesondere die Hülse gestaucht oder das Keilelement axial verschoben werden.

Ein Ausführungsbeispiel der Erfindung wird nachfolgend anhand der Figuren näher erläutert.

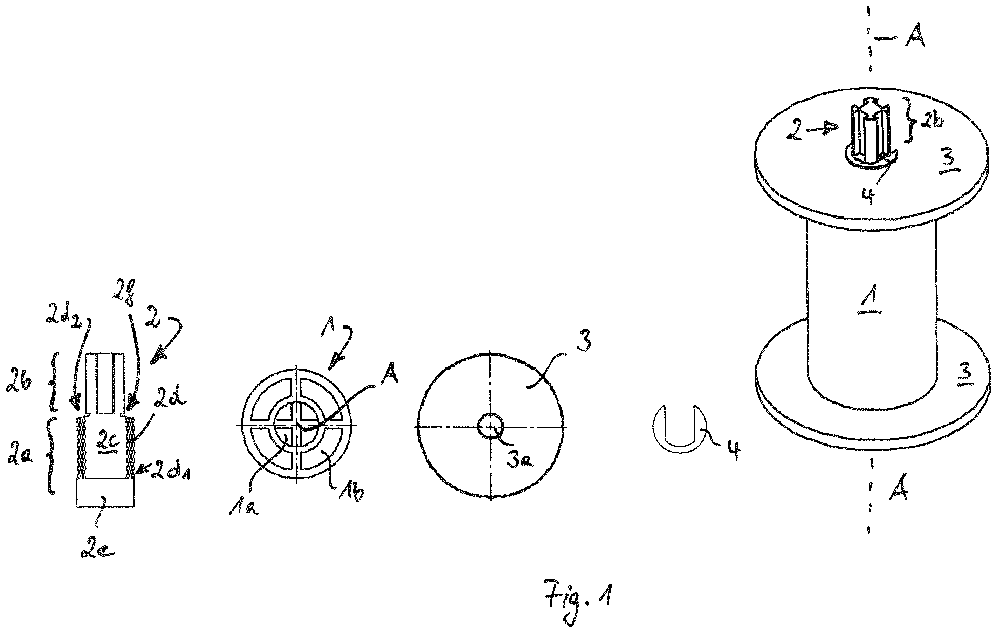

Die Figur 1 zeigt eine Übersicht über die Bestandteile des Wickelsystems.

Es umfasst einen Wickelkern 1, auf den hier nicht gezeigte Hohlfasern bzw. Hohlfasermatten aufwickelbar sind. Der Wickelkern 1 hat eine zentrale um die Wickelachse A angeordnete Ausnehmung 1a, vorzugsweise in seinen beiden gegenüberliegenden Stirnflächen, vorzugsweise, die sich jeweils ausgehend von der Stirnfläche in den Wickelkern hinein erstreckt. Die Ausnehmung ist durch einen Kanal im Wickelkern gebildet. Die Ausnehmung 1a ist weiterhin von einem Kanal 1b koaxial umgeben, insbesondere mit radialen Streben zwischen den Wandungen beider Kanäle 1a, 1b. Eine solche Ausführung ist nicht zwingend nötig.

In die hier mit kreisrundem Querschnitt ausgebildeten Ausnehmung 1a kann ein Kupplungselement 2 eingesteckt werden. Dieses hat einen in die Ausnehmung 1a passenden ersten Verbindungsbereich 2a und einen zweiten Verbindungsbereich 2b, der vorgesehen ist zur Verbindung mit einem hier nicht weiter gezeigten Antrieb. Dieser zweite Verbindungsbereich ist somit eingerichtet, um drehfest mit dem Antrieb, z.B. einer Kupplung des Antriebs verbunden zu werden.

Das Kupplungselement 2 weist weiterhin einen Schaftbereich 2c auf, um den herum als Spreizelement eine Hülse 2d aus einem Elastomer, z.B. aus Silikon angeordnet ist. Am freien Ende des ersten Verbindungsbereiches 2a liegt die Hülse mit einem ersten axialen Ende 2d1 an einer Kraftausübungsfläche 2e des Kupplungselementes an. Das zweite axiale Ende 2d2 der Hülse 2d ist frei. Durch axiales Stauchen der Hülse 2d, also eine Kraftausübung, welche die axialen Enden 2d1 und 2d2 aufeinander zu bewegt kann die Hülse 2d radial verdickt werden.

Das Wickelsystem kann weiterhin ein Hilfselement 3 umfassen, das als Scheibe 3 ausgebildet sein kann. Diese hat eine innere Öffnung 3a, die über den zweiten Verbindungsbereich 2b des Kupplungselementes 2 herüber geführt werden kann. Der Außendurchmesser der Scheibe 3 ist größer als der Außendurchmesser des Wickelkerns 1.

Das Wickelsystem umfasst in einer möglichen Ausgestaltung auch einen Sicherungsring 4, der in eine Ringnut 2f zwischen den Verbindungsbereichen 2a und 2b des Kupplungselementes 2 eingesetzt werden kann.

Rechts in der Figur 1 ist das zusammengesetzte Wickelsystem abgebildet, nachdem das Kupplungselement 2 in die Ausnehmung 1a des Wickelkerns 1 eingesetzt wurde und die Scheibe 3 über das Kupplungselement 2 herübergeführt und auf die Stirnfläche des Wickelkerns 1 aufgelegt wurde.

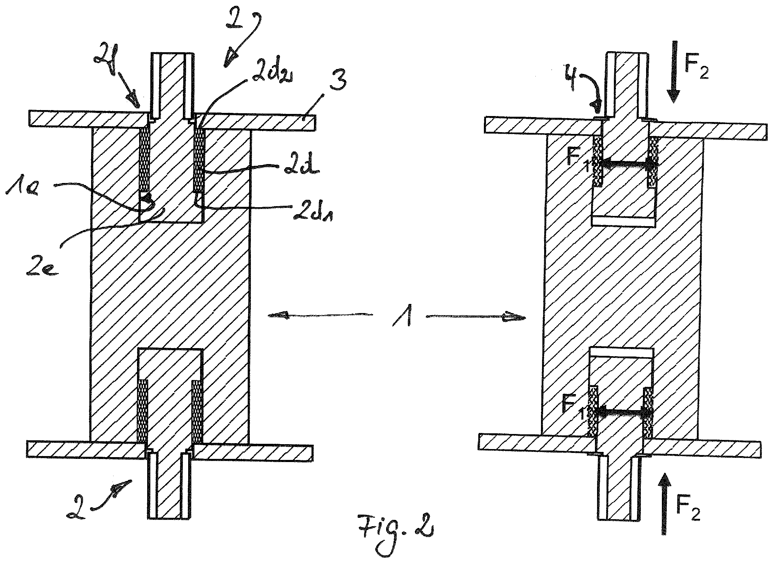

Die Figur 2 zeigt im Schnitt das zusammengebaute Wickelsystem in zwei Zuständen.

Links in der Figur 2 ist zu erkennen, dass das Kupplungselement 2 in die Ausnehmung 1a des Wickelkerns 1 eingesetzt ist, ohne dass das Spreizelement 2d, hier die Hülse 2d aufgespreizt, also verdickt ist. Die Scheibe 3 liegt auf der Stirnfläche des Wickelkerns 1 auf und kann vorzugsweise bereits im nicht verspannten Zustand die zweite axialen Stirnfläche 2d2 der Hülse 2d kontaktieren. Dafür liegt dann das zweite axiale Ende 2d2 der Hülse in der Ebene der Stirnfläche des Wickelkerns. Das zweite axiale Ende 2d2 der Hülse kann alternativ auch unter der Ebene der Stirnfläche des Wickelkerns liegen und das Hilfselement anfänglich nicht kontaktieren.

Die Ringnut 2f des Kupplungselementes 2 liegt unter der Oberfläche der Scheibe 3 und ist nicht zugänglich für den Sicherungsring 4.

Der Verbindungsbereich 2a liegt vollständig, aber nicht verbunden im Wickelkern 1. Der Verbindungsbereich 2b liegt außerhalb des Wickelkerns 1 und ist zum Ankoppeln an einen nicht gezeigten Antrieb zugänglich.

Zur Befestigung des Kupplungselementes 2 im Wickelkern 1 wird die Hülse gespreizt, was dadurch erfolgt, dass das Kupplungselement bereichsweise aus der Ausnehmung herausgezogen wird, zumindest bis dass die Ringnut über der Scheibe 3 zugänglich wird. Hierdurch wird die Kraftausübungsfläche 2e in Richtung zur Scheibe 3 bewegt. Da die Hülse 2d zwischen der Kraftausübungsfläche 2e und der Scheibe 3 eingeschlossen ist, wird durch die Bewegung der Kraftausübungsfläche 2e die Hülse 2d axial gestaucht und verdickt sich dadurch. Hierdurch wird die Mantelfläche der Hülse 2d an die Innenfläche der Ausnehmung 1a angepresst, was einen Kraftschluß mit der radial wirkenden Kraft F1 erzeugt gemäß der Darstellung rechts in der Figur 2.

Rechts in der Figur 2 ist der Sicherungsring 4 in die Ringnut 2f eingesetzt, so dass das Kupplungselement 2 nicht wieder in die Ausnehmung 1a zurückweichen kann. Dadurch wirkt die Kraft F1 statisch fort und das Kupplungselement sitzt in der Ausnehmung 1a des Wickelkerns 1 fest.

Es wirkt weiterhin eine Kraft F2 die dadurch entsteht, dass die Hülse 2d aufgrund ihrer axialen Kompression das Kupplungselement 2 in die Ausnehmung 1a zurückzieht, dies aber durch die Abstützung des Sicherungsringes 4 auf der Scheibe 3 nicht möglich ist. Die Kraft F2 wirkt somit über den Sicherungsring 4 auf die Scheibe 3 und drückt diese zusätzlich auf die Stirnfläche des Wickelkerns 1.

Im gespreizten Zustand des rechten Teils der Figur 2 kann der Wickelkern 1 beidseitig mit einem Antrieb verbunden werden, um die wenigstens eine Hohlfasermatte auf den Wickelkern aufzuwickeln.

Nachdem das erfolgt ist kann der Wickelkern vom Antrieb getrennt werden, der Sicherungsring 4 vom jeweiligen Kupplungselement 2 abgezogen und diese zusammen mit der Scheibe 3 vom Wickelkern 1 entfernt werden.

Der Wickelkern kann dann mit den getragenen Hohlfasern in das Gehäuse einer Austauschvorrichtung eingesetzt und die Hohlfasern damit verpottet/vergossen werden. Der Kanal 1a und/oder 1b im Wickelkern kann vorzugsweise zum Transport von Medium, z.B. Blut durch den Wickelkern genutzt werden.

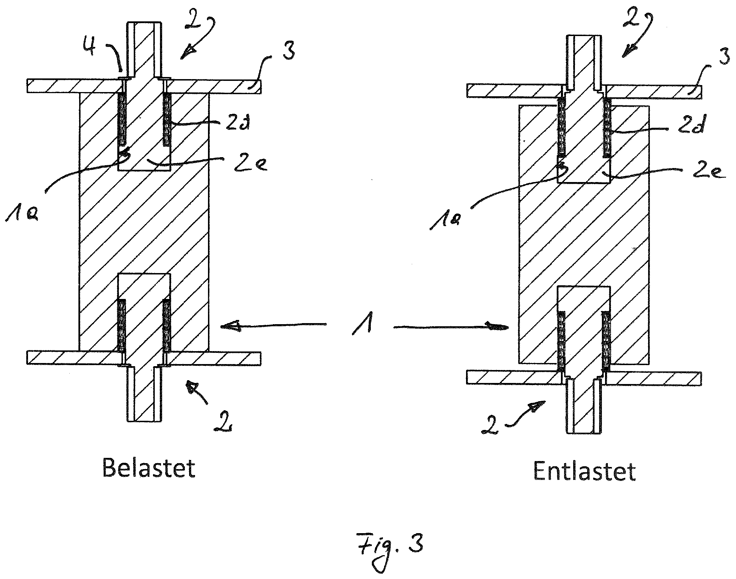

Die Figur 3 zeigt rechts eine Ausführung, bei welcher im ungespreizten Zustand die Hülse 2d nach Einsetzen des Kupplungselementes 2 in den Wickelkern 1 mit dem axialen Ende 2d2 über die Stirnfläche des Wickelkerns 1 übersteht und somit durch Aufsetzen der Scheibe 3 als Hilfselement die Hülse axial gestaucht wird, wodurch diese sich verdickt und die kraftschlüssige Verbindung entsteht. Links in der Figur 3 ist die axiale Sicherung der so erzielten Lage mit dem Sicherungsring 4 gezeigt, wie bei der Figur 2 rechts. Im Übrigen entspricht die Figur 3 der Ausführung von Figur 2

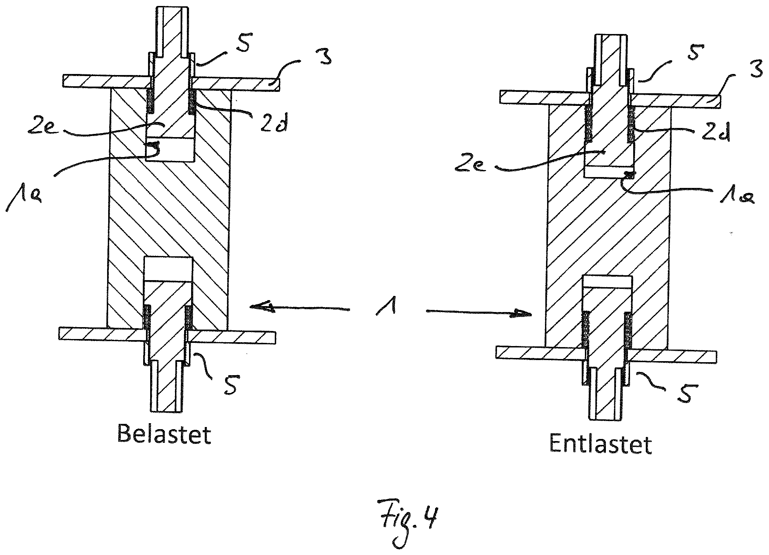

Die Figur 4 zeigt eine alternative Ausführung, um die Befestigung des Kupplungselementes 2 zu bewirken.

Das Kupplungselement 2 weist zwischen dem Verbindungsbereich 2b für die Verbindung mit einem Antrieb und dem axialen Ende 2d2 der Hülse 2d einen Außengewindeabschnitt auf, auf den ein Ring 5 oder eine Mutter 5 mit entsprechendem Innengewinde aufgedreht werden kann, z.B. nachdem die Scheibe 3 über den Verbindungsbereich 2b herüber und auf die Stirnfläche des Wickelkerns 1 aufgelegt wurde. Nachdem der Ring 5 beim Aufdrehen die Scheibe 3 kontaktiert hat zieht der Ring 5 das Kupplungselement 2 aus dem Wickelkern in axialer Richtung bereichsweise heraus, wodurch die Hülse 2d, die mit ihrem oberen axialen Ende 2d2 an der Scheibe 3 anliegt, axial gestaucht und radial aufgespreizt / verdickt wird. Hierdurch wird die kraftschlüssige Verbindung hergestellt. Rechts in der Figur 4 ist der nicht gespreizte Zustand gezeigt und links der gespreizte.

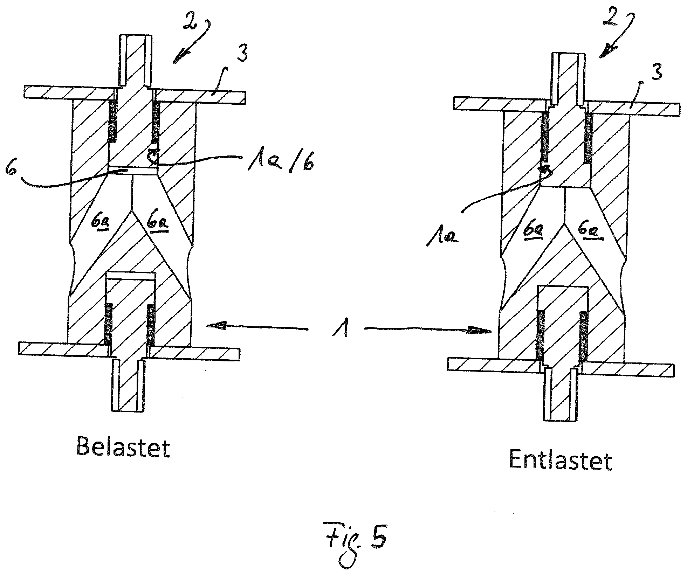

Die Figur 5 zeigt eine mögliche Ausführung, die unabhängig von den Arten der möglichen Spreizung gewählt sein kann. Beispielhaft gezeigt ist die Spreizung wie in Figur 2. Bezogen auf die Figur 5 ist die obere Ausnehmung 1a im Wickelkern 1 als ein Kanal 6 ausgebildet, der sich von der oberen Stirnfläche des Wickelkerns 1 ausgehend in diesen hinein erstreckt und in Kanalabschnitte 6a aufzweigt, die in die Mantelfläche des Wickelkerns 1 münden. So können Hohlfasern von radial innen mit einem durch diesem Kanal geführten Medium angeströmt werden. Links in der Figur 5 ist das Kupplungselement 2 gespreizt und rechts ungespreizt.

## Patentansprüche

1. Wickelsystem zur Durchführung des Aufwickelns von verketteten Hohlfasern, insbesondere von stoff- und/oder energiepermeablen Hohlfasern, auf einen Wickelkern (1) umfassend:
a. einen Wickelkern (1) einer Austauschvorrichtung für den Stoff- und/oder Wärmeaustausch zwischen zwei Medien, insbesondere eines Oxygenators und/oder Wärmetauschers, auf den verkettete Hohlfasern aufwickelbar sind und der vorgesehen, insbesondere eingerichtet ist, zusammen mit den auf ihn aufgewickelten Hohlfasern in das Gehäuse der Vorrichtung eingesetzt zu werden und darin zu verbleiben,
**gekennzeichnet durch**
b. wenigstens ein Kupplungselement (2), vorzugsweise zwei Kupplungselemente (2), wobei das wenigstens eine Kupplungselement (2) zwei sich axial gegenüberliegende Verbindungsbereiche (2a, 2b) aufweist und temporär mit dem ersten Verbindungsbereich (2a) mit dem Wickelkern (1) drehfest verbindbar ist und mit dem zweiten Verbindungsbereich (2b) mit einem Antrieb drehfest verbindbar ist, mittels dem der Wickelkern (1) um eine Wickelachse (A) rotierbar ist,
wobei
c. der erste Verbindungsbereich (2a) des Kupplungselementes (2) ein Spreizelement (2d) umfasst, insbesondere dessen Außenquerschnitt durch Aufspreizen vergrößerbar ist, und der Wickelkern (1) in wenigstens einer axialen Stirnfläche, vorzugsweise in beiden axialen Stirnflächen eine um die Wickelachse (A) angeordnete Ausnehmung (1a) aufweist, in welche der erste Verbindungsbereich (2a) eines Kupplungselementes (2) hineinsteckbar und durch Aufspreizen des Spreizelementes (2d) der Wickelkern (1) mit dem Kupplungselement (2) drehfest verbindbar ist.

2. Wickelsystem nach Anspruch 1, **dadurch gekennzeichnet, dass** das wenigstens eine Kupplungselement (2) an seinem ersten Verbindungsbereich (2a) einen Schaft aufweist der in axialer Richtung geteilt ist in wenigstens zwei axial parallel nebeneinanderliegende Schaftteile zwischen denen als Spreizelement ein axial zwischen die Schaftteile bewegliches Keilelement angeordnet ist.

3. Wickelsystem nach Anspruch 1, **dadurch gekennzeichnet, dass** das wenigstens eine Kupplungselement (2) an seinem ersten Verbindungsbereich einen Schaft mit einem Schaftabschnitt (2c) aufweist, um den herum als Spreizelement eine Hülse (2d) aus einem elastischen Material, insbesondere aus einem Elastomer, vorzugsweise aus einem Vulkanisat von Silikonkautschuk oder Naturkautschuk, angeordnet ist, wobei die Hülse (2d) durch axiale Bewegung ihrer Stirnflächen (2d1, 2d2) zueinander, insbesondere durch Einwirkung einer Kraft in axialer Richtung auf die ringförmigen Stirnflächen (2d1, 2d2) der Hülse (2d), in radialer Richtung in seiner Größe änderbar ist.

4. Wickelsystem nach Anspruch 3, **dadurch gekennzeichnet, dass** die Hülse (2d) mit einem ersten Ende (2d1) ihrer beiden axialen Enden (2d1, 2d2) an einer zum Schaftbereich (2c) ortsfesten ersten Kraftausübungsfläche (2e) des Kupplungselementes (2) anliegt und die Hülse (2d) mit dem zweiten axialen Ende (2d2) an einer zweiten Kraftausübungsfläche des Kupplungselementes (2) anliegt, wobei die zweite Kraftausübungsfläche relativ zur ersten Kraftausübungsfläche (2e) in axialer Richtung der Hülse (2d), insbesondere auf dem Schaft beweglich ist, vorzugsweise mittels eines am Kupplungselement (2) vorgesehenen Betätigungselementes.

5. Wickelsystem nach einem der vorherigen Ansprüche, **dadurch gekennzeichnet, dass** in einem Zustand der Aufspreizung die Position des Kupplungselementes (2) relativ zum Wickelkern (1) und zu einem zum Wickelkern (1) separaten Hilfselement (3), das nach Einsetzen des ersten Verbindungsbereiches (2a) des Kupplungselementes (2) in die Ausnehmung (1a) des Wickelkerns (1) über das Kupplungselement (2) herüber auf eine axiale Stirnfläche des Wickelkerns (1) aufsetzbar ist, axial fixierbar ist.

6. Wickelsystem nach Anspruch 5, **dadurch gekennzeichnet, dass** das Hilfselement (3), durch eine über den Wickelkern (1) im Querschnitt / Durchmesser in radialer Richtung überstehenden Scheibe (3) ausgebildet ist, insbesondere mittels der beim Wickeln die Hohlfasermatte relativ zum Wickelkern (1) positionierbar ist.

7. Wickelsystem nach Anspruch 5 oder 6, **dadurch gekennzeichnet, dass** das Hilfselement (3) für die Hülse (2d) eine zweite Kraftausübungsfläche bildet, die von der Hülse (2d) mit dem zweiten axialen Ende (2d2), insbesondere dem zum zweiten Verbindungsbereich (2b) des Kupplungselementes (2) weisenden Ende der Hülse (2d), kontaktierbar ist, wobei
a. das zum zweiten Verbindungsbereich (2b) des Kupplungselementes (2) weisende zweite Ende (2d2) der Hülse (2d) im in den Wickelkern (1) eingesetzten Zustand des Kupplungselementes (2) über die axiale Stirnfläche des Wickelkernes (1) hervorsteht und die Kraftausübung auf die Hülse (2d) durch das Aufsetzen des Hilfselementes (3) bewirkbar ist, insbesondere wodurch das zweite Ende (2d2) der Hülse (2d) in Richtung zum ersten Ende (2d1) der Hülse (2d) verlagerbar ist, oder
b. das zum zweiten Verbindungsbereich (2b) des Kupplungselementes (2) weisende zweite Ende (2d2) der Hülse (2d) im in den Wickelkern (1) eingesetzten Zustand des Kupplungselementes (2) unter der axialen Stirnfläche oder in der Ebene der axialen Stirnfläche des Wickelkernes (1) liegt und die Kraftausübung / das Aufspreizen nach Aufsetzen des Hilfselementes (3) durch eine axiale Bewegung des Kupplungselements (2), insbesondere durch eine aus dem Wickelkern (1) heraus gerichtete Bewegung bewirkbar ist, insbesondere wodurch das erste Ende (2d1) der Hülse (2d) in Richtung zum zweiten Ende (2d2) der Hülse (2d) verlagerbar ist.

8. Wickelsystem nach einem der vorherigen Ansprüche 5 bis 7, **dadurch gekennzeichnet, dass** die Position des Kupplungselementes (2) axial fixierbar ist mittels einem in eine Ringnut (2f) am Kupplungselement (2) einsetzbaren Sicherungsring (4), mit dem das Kupplungselement (2) an der Oberfläche des Hilfselementes (3) abstützbar ist.

9. Wickelsystem nach einem der vorherigen Ansprüche, **dadurch gekennzeichnet, dass** am Kupplungselement (2) ein Betätigungselement (5) vorgesehen ist, mit dem die Aufspreizung des Spreizelementes (2d) im ersten Verbindungsbereich (2a) und/oder eine axiale Fixierung des Kupplungselementes (2) bewirkbar ist, insbesondere das sich an dem Hilfselement (3) oder einem die zweite Kraftausübungsfläche aufweisenden Element oder einem am Kupplungselement (2) angeordnete Stützelement abstützt, insbesondere wobei das Betätigungselement (5) ausgebildet ist als eine Mutter auf einem Gewindeabschnitt des Kupplungselementes (2), welcher mit dem Spreizelement (2d) verbunden ist oder als ein mit einem Hebel bewegbarer Exzenter, der um eine Achse drehbar ist.

10. Wickelsystem nach einem der vorherigen Ansprüche, **dadurch gekennzeichnet, dass** eine Ausnehmung (1a) im Wickelkern (1) für die Aufnahme des Kupplungselementes (2) gebildet ist durch einen den Wickelkern (1) zumindest bereichsweise durchdringenden Kanal, der nach Einsetzen des Wickelkernes (1) in das Gehäuse der Stoffaustauschvorrichtung von einem Medium durchströmbar ist, insbesondere von Blut.

11. Wickelsystem nach einem der vorherigen Ansprüche, **dadurch gekennzeichnet, dass** eine Ausnehmung (1a) im Wickelkern für die Aufnahme des Kupplungselementes (2) umgeben ist von einem zur Ausnehmung (1a) zumindest bereichsweise koaxial verlaufenden Kanal (1b), der nach Einsetzen des Wickelkernes (1) in das Gehäuse der Stoffaustauschvorrichtung von einem Medium durchströmbar ist, insbesondere von Blut.

## Claims

1. Winding system for winding concatenated hollow fibers, in particular mass-permeable and/or energy-permeable hollow fibers, onto a winding core (1), the system comprising:
a. a winding core (1) of a transfer device for the mass transfer and/or heat exchange between two media, in particular of an oxygenator and/or a heat exchanger, onto which winding core concatenated hollow fibers can be wound and which winding core is provided, in particular set up, to be inserted, together with the hollow fibers wound thereon, into the housing of the device and to remain therein,
**characterised by**
b. at least one coupling element (2), preferably two coupling elements (2), wherein the at least one coupling element has two axially opposing connecting regions (2a, 2b) and is temporarily connectable, by means of the first connecting region(2a), with the winding core (1) for conjoint rotation therewith and is temporarily connectable by means of the second connecting region (2b), with a drive for conjoint rotation therewith, the drive being provided for the rotation of the winding core (1) about a winding axis (A),
wherein
the first connecting region (2a) of the coupling element (2) comprises an expansion element (2d), in particular an expansion element the outer cross section of which can be increased by expansion, and the winding core (1) has in at least one axial end face, preferably in both axial end faces, a recess (1a) around the winding axis (A), into which recess the first connecting region (2a) of a coupling element (2) can be inserted and in which the winding core (1) can, by means of expansion of the expansion element (2d) be connected to the coupling element (2) for conjoint rotation therewith.

2. Winding system according to claim 1, **characterized in that** the at least one coupling element (2) has at its first connecting region (2a) a shank which is split axially into at least two axially parallel adjoining shank parts between which a wedge element is provided as an expanding component, which wedge element can be moved axially between the shank parts.

3. Winding system according to claim 1, **characterized in that** the at least one coupling element (2) has at its first connecting region a shank with a shank section (2c) around which a sleeve (2d) made of an elastic material, in particular an elastomer, preferably a vulcanizate of silicone rubber or natural rubber, is arranged as an expansion element, wherein the sleeve (2d) can be changed in its size in radial direction as a result of axial movement of the annular end faces (2d1, 2d2) of the sleeve (2d) towards each other, in particular through the application of an axial force on the annular end faces (2d1, 2d2) of the sleeve (2d).

4. Winding system according to claim 3, **characterized in that** the sleeve (2d) with a first end (2d1) of its two axial ends (2d1, 2d2) contacts a first area of force application (2e) of the coupling element (2), which area is stationary in relation to the shank area (2c), and the sleeve (2d) with the second axial end (2d2) contacts a second area of force application of the coupling element (2), wherein the second area of force application is movable relative to the first area of force application (2e) in axial direction of the sleeve (2d), in particular being movable on the shank, preferably by means of an actuating element provided at the coupling element (2).

5. Winding system according to any of the preceding claims, **characterized in that** in one state of expansion, the position of the coupling element (2) can be axially fixed relative to the winding core (1) and relative to an auxiliary element (3) separate from the winding core (1) which auxiliary element (3) can, after the first connecting region (2a) of the coupling element (2) has been inserted into the recess (1a) of the winding core (1), be pushed over the coupling element (2) and placed onto an axial end face of the winding core (1).

6. Winding system according to claim 5, **characterized in that** the auxiliary element (3) is formed by a disc (3) protruding radially beyond the cross section / diameter of the winding core (1), in particular a disc which can be used to position the hollow fiber mat relative to the winding core (1) during the winding.

7. Winding system according to claim 5 or 6, **characterized in that** the auxiliary element (3) for the sleeve (2d) forms a second area of force application which can be contacted by the sleeve (2d) with the second axial end (2d2), in particular the end of the sleeve (2d) pointing to the second connecting region (2b) of the coupling element (2), wherein
a. the second end (2d2) of the sleeve (2) pointing to the second connecting region (2b) of the coupling element (2) protrudes beyond the axial end face of the winding core (1) when the coupling element (2) is in its state of insertion in the winding core (1), and the application of the force on the sleeve (2d) can be caused by placing the auxiliary element (3), in particular as a result of which, the second end (2d2) of the sleeve (2) can be displaced in the direction of the first end (2d1) of the sleeve (2), or
b. the second end (2d2) of the sleeve (2d) pointing to the second connecting area (2b) of the coupling element (2) lies below the axial end face or in the plane of the axial end face of the winding core (1) when the coupling element (2) is in its state of insertion in the winding core (1), and the application of the force / the expansion after the placement of the auxiliary element (3) can be caused by an axial movement of the coupling element (2), in particular a movement directed out from the winding core (1), in particular as a result of which, the first end (2d1) of the sleeve (2d) can be displaced in the direction of the second end (2d2) of the sleeve (2d).

8. Winding system according to any of the preceding claims 5 to 7, **characterized in that** the position of the coupling element (2) can be axially fixed by means of a retaining ring (4) that can be inserted into an annular groove (2f) at the coupling element (2) and that can support the coupling element (2) at the surface of the auxiliary element (3).

9. Winding system according to any of the preceding claims, **characterized in that** at the coupling element (2), an actuating element (5) is provided which can cause an expansion of the expansion element (2d) in the first connecting region (2a) and/or an axial fixing of the coupling element (2), in particular an actuating element (5) supported by the auxiliary element (3) or by an element exhibiting the second area of force application or by a supporting element arranged at the coupling element (2), in particular wherein the actuating element (5) designed as a nut on a threaded section of the coupling element (2) which is connected with the expansion element (2d) or as an eccentric rotatable about an axis with a lever.

10. Winding system according to any of the preceding claims, **characterized in that** a recess (1a) in the winding core (1) for receiving the coupling element (2) is formed by a channel that at least partly penetrates the winding core (1), through which channel, after the insertion of the winding core (1) into the housing of the mass transfer device, a medium can flow, in particular blood.

11. Winding system according to any of the preceding claims, **characterized in that** a recess (1a) in the winding core for receiving the coupling element (2) is surrounded by a channel (1b) which at least in parts runs coaxial in relation to the recess (1a) and through which channel, after insertion of the winding core (1) into the housing of the mass transfer device, a medium can flow, in particular blood.

## Revendications

1. Système d'enroulement pour réaliser l'enroulement de fibres creuses reliées entre elles, notamment de fibres creuses perméables aux matières et/ou à l'énergie, sur un noyau d'enroulement (1), comprenant :
a. un noyau d'enroulement (1) d'un dispositif d'échange pour l'échange de matière et/ou de chaleur entre deux milieux, notamment d'un oxygénateur et/ou d'un échangeur de chaleur, sur lequel peuvent être enroulées des fibres creuses reliées entre elles et qui est prévu, notamment adapté, pour être inséré conjointement avec les fibres creuses enroulées sur lui dans le boîtier du dispositif et pour y rester,
**caractérisé par**
b. au moins un élément d'accouplement (2), de préférence deux éléments d'accouplement (2), l'au moins un élément d'accouplement (2) présentant deux zones de liaison (2a, 2b) axialement opposées et pouvant être relié temporairement de manière solidaire en rotation au noyau d'enroulement (1) par la première zone de liaison (2a) et pouvant être relié de manière solidaire en rotation à un entraînement par la deuxième zone de liaison (2b), au moyen duquel le noyau d'enroulement (1) peut tourner autour d'un axe d'enroulement (A),
où
c. la première zone de liaison (2a) de l'élément d'accouplement (2) comprend un élément d'écartement (2d), notamment dont la section transversale extérieure peut être agrandie par écartement, et le noyau d'enroulement (1) présente, dans au moins une surface frontale axiale, de préférence dans les deux surfaces frontales axiales, un évidement (1a) disposé autour de l'axe d'enroulement (A), dans lequel la première zone de liaison (2a) d'un élément d'accouplement (2) peut être enfichée et, par écartement de l'élément d'écartement (2d), le noyau d'enroulement (1) peut être relié de manière solidaire en rotation à l'élément d'accouplement (2).

2. Système d'enroulement selon la revendication 1, **caractérisé en ce que** l'au moins un élément d'accouplement (2) présente, au niveau de sa première zone de liaison (2a), une tige qui est divisée dans la direction axiale en au moins deux parties de tige parallèles entre lesquelles est agencé, en tant qu'élément d'écartement, un élément en coin mobile axialement entre les parties de tige.

3. Système d'enroulement selon la revendication 1, **caractérisé en ce que** l'au moins un élément d'accouplement (2) présente, au niveau de sa première zone de liaison, une tige avec une partie de tige (2c) autour de laquelle est agencée, en tant qu'élément d'écartement, une douille (2d) en un matériau élastique, notamment en un élastomère, de préférence en un vulcanisat de caoutchouc silicone ou de caoutchouc naturel, la douille (2d) pouvant être modifiée en termes de sa taille dans la direction radiale par mouvement axial de ses surfaces frontales (2d1, 2d2) l'une par rapport à l'autre, notamment sous l'effet d'une force dans la direction axiale sur les surfaces frontales annulaires (2d1, 2d2) de la douille (2d).

4. Système d'enroulement selon la revendication 3, **caractérisé en ce que** la douille (2d) s'appuie par une première extrémité (2d1) de ses deux extrémités axiales (2d1, 2d2) contre une première surface d'application de force (2e) de l'élément d'accouplement (2), qui est fixe par rapport à la zone de tige (2c), et la douille (2d) s'appuie par sa deuxième extrémité axiale (2d2) contre une deuxième surface d'application de force de l'élément d'accouplement (2), la deuxième surface d'application de force étant mobile par rapport à la première surface d'application de force (2e) dans la direction axiale de la douille (2d), notamment sur la tige, de préférence au moyen d'un élément d'actionnement prévu sur l'élément d'accouplement (2).

5. Système d'enroulement selon l'une quelconque des revendications précédentes, **caractérisé en ce que**, dans un état d'écartement, la position de l'élément d'accouplement (2) par rapport au noyau d'enroulement (1) et à un élément auxiliaire (3) séparé du noyau d'enroulement (1), qui, après insertion de la première zone de liaison (2a) de l'élément d'accouplement (2) dans l'évidement (1a) du noyau d'enroulement (1), peut être placé sur une surface frontale axiale du noyau d'enroulement (1) par l'intermédiaire de l'élément d'accouplement (2), peut être fixée axialement.

6. Système d'enroulement selon la revendication 5, **caractérisé en ce que** l'élément auxiliaire (3) est réalisé par un disque (3) dépassant du noyau d'enroulement (1) dans la direction radiale en termes de section transversale/diamètre, notamment au moyen duquel le mat de fibres creuses peut être positionné par rapport au noyau d'enroulement (1) pendant l'enroulement.

7. Système d'enroulement selon la revendication 5 ou 6, **caractérisé en ce que** l'élément auxiliaire (3) forme pour la douille (2d) une deuxième surface d'application de force qui peut être mise en contact avec la douille (2d) par la deuxième extrémité axiale (2d2), notamment l'extrémité de la douille (2d) orientée vers la deuxième zone de liaison (2b) de l'élément d'accouplement (2),
a. la deuxième extrémité (2d2) de la douille (2d) orientée vers la deuxième zone de liaison (2b) de l'élément d'accouplement (2) faisant saillie, à l'état de l'élément d'accouplement (2) inséré dans le noyau d'enroulement (1) de la surface frontale axiale du noyau d'enroulement (1) et l'application de force sur la douille (2d) pouvant être effectuée par la mise en place de l'élément auxiliaire (3), ce qui permet notamment de déplacer la deuxième extrémité (2d2) de la douille (2d) en direction de la première extrémité (2d1) de la douille (2d), ou
b. la deuxième extrémité (2d2) de la douille (2d) orientée vers la deuxième zone de liaison (2b) de l'élément d'accouplement (2) se situant, à l'état de l'élément d'accouplement (2) inséré dans le noyau d'enroulement (1), sous la surface frontale axiale ou dans le plan de la surface frontale axiale du noyau d'enroulement (1) et l'application de force/l'écartement après la mise en place de l'élément auxiliaire (3) pouvant être effectué par un mouvement axial de l'élément d'accouplement (2), notamment par un mouvement dirigé hors du noyau d'enroulement (1), ce qui permet notamment de déplacer la première extrémité (2d1) de la douille (2d) en direction de la deuxième extrémité (2d2) de la douille (2d).

8. Système d'enroulement selon l'une quelconque des revendications 5 à 7 précédentes, **caractérisé en ce que** la position de l'élément d'accouplement (2) peut être fixée axialement au moyen d'une bague de sécurisation (4) pouvant être insérée dans une rainure annulaire (2f) sur l'élément d'accouplement (2), avec laquelle l'élément d'accouplement (2) peut être soutenu sur la surface de l'élément auxiliaire (3).

9. Système d'enroulement selon l'une quelconque des revendications précédentes, **caractérisé en ce qu'**un élément d'actionnement (5) est prévu sur l'élément d'accouplement (2), avec lequel l'écartement de l'élément d'écartement (2d) dans la première zone de liaison (2a) et/ou une fixation axiale de l'élément d'accouplement (2) peuvent être effectués, notamment qui est soutenu sur l'élément auxiliaire (3) ou sur un élément présentant la deuxième surface d'application de force ou sur un élément de soutien agencé sur l'élément d'accouplement (2), l'élément d'actionnement (5) étant notamment réalisé sous la forme d'un écrou sur une section filetée de l'élément d'accouplement (2), qui est relié à l'élément d'écartement (2d), ou sous la forme d'un excentrique mobile à l'aide d'un levier, qui peut tourner autour d'un axe.

10. Système d'enroulement selon l'une quelconque des revendications précédentes, **caractérisé en ce qu'**un évidement (1a) est formé dans le noyau d'enroulement (1) pour recevoir l'élément d'accouplement (2) par un canal traversant au moins par zones le noyau d'enroulement (1), qui peut être traversé par un fluide, notamment du sang, après l'insertion du noyau d'enroulement (1) dans le boîtier du dispositif d'échange de matière.

11. Système d'enroulement selon l'une quelconque des revendications précédentes, **caractérisé en ce qu'**un évidement (1a) dans le noyau d'enroulement pour recevoir l'élément d'accouplement (2) est entouré d'un canal (1b) s'étendant au moins par zones de manière coaxiale par rapport à l'évidement (1a), lequel peut être traversé par un fluide, notamment du sang, après insertion du noyau d'enroulement (1) dans le boîtier du dispositif d'échange de matière.
